# EUROPEAN PATENT APPLICATION

(11) **EP 1 177 805 A1**
(43) Date of publication of application: **06.02.2002**
(21) Application number: 00116509.1
(22) Date of filing: 31.07.2000
(51) Int. Cl.: A61M 15/00

(54) **Powder inhaler**

(71) Applicant: Maryland Financial Inc., Road Town-Tortola, British Virgin Islands (GB)
(72) Inventor: Pera, Ivo, 33026 Hollywood (FL) (US)
(74) Representative: Turini, Laura

(57) **Abstract**

This invention consists of a *Mono-dose Inhaling Device,* disposable or not, for the inhalation of powdered medicaments, formed by a body (1) connected to a base (14) and a cover member (4) comprising a pointed element (12) that passes through a spring (3). Under this spring, a tablet is placed on a suitable housing portion (10) over a grid reservoir (5) placed at one end of the chamber (15) of the body (1). Once the tablet (2) is put on its portion (10) and the body (1) is closed with the cover (4), it's sufficient to place the opening (8) into the mouth, press the cover (4) and breathe in, so that the powdered substance dispersed into the chamber (15) can reach the lungs. Said inhaler may be used with specific accessories also for inhaling the substances by the nose or for a deep inhalation of the powdered medicaments.

## Description

### Technical Field

The present invention relates to a *Mono-dose Inhaler* to use in Respiratory Therapy and other Diseases through the topical administration of Medications through the mucous linings of tracheo-bronchial tree and the lungs.

### Background art

As the lung is considered to be one of the more effective, noninvasive route of administration to the systemic circulation, a number of powdered medications can be used to treat a variety of conditions that accompany a lot of different diseases. Conditions requiring an inhaler, particularly in such therapy where can be administered to a patient on a program of home care, include: (I) infection, (2) mucous edema, (3) tenacious secretions, (4) foam buildup, (5) bronchosplasms, and (6) loss of compliance.

Many useful medicaments, and especially penicillin and related antibiotics, are subject to substantial or, in some instances, complete alteration by the stomach juices when administered orally. Different patients will react differently to the same dosage at different times. On this account, in past oral dosages was supplemented by checking blood samples to ascertain how much medicament has found its way into the blood stream, or by parental administration.

Powdered medicaments administered by inhalation are not frequently used for delivery into the systemic circulation, because of various factors that contribute to erratic or difficult-to-achieve blood levels. Whether or not the powder drugs reaches and is retained in pulmonary alveoli depends critically upon particle size. The literature reports that the optimum particle size for penetration into the pulmonary cavity is of the order of 1/2 to 7 um.

The general properties given rise to errors of such inhalers in dispensing proper Medicaments are specific directional and velocity characteristics including instability and concentration of the mass of particles administered in a unit volume of air.

Moreover in a definitive clinical setting the patient's inhaling flow can vary significantly, during my research and development I have considered appropriate to test several types of Inhalers with different flow rate in order to develop a novel *Mono-dose Inhaling Device,* which is obviating the above described disadvantage, by effectively and smoothly inhaling the powdered medicaments into the pulmonary tract by the simple inspiration of air by a patient.

Several devices have been developed for the administration of micronized powders of relatively potent drugs. The Norisodrine Sulfate Aerohaler Cartridge (***Abbott***) is an example of such product. In the use of this Aereohaler, inhalation by the patient causes a small ball to strike a cartridge containing the drug. The force of the ball shakes the proper amount of the powder free, permitting its inhalation. Another device, the Spinhaler (***Fisons***), TurboHaler from Astra are propeller-driven device designed to deposit a mixture of lactose and micronized cromolyn sodium into the lung, other devices developed by ***Glaxo*,** such as Rotahaler, Beclodisk, Diskhaler, Ventodisk and Diskus, all for the administration of powdered drugs for the Respiratory Tract (like Ventolin, Albuterol, Salbritanol, Serevent, etc.) and several other types of these inhalators at present on the market, however most are cumbersome, complicated and expensive.

Consequently, these multi-dose devices have to be reused, whereas for reasons of hygiene and, particularly owing to the difficulty of keeping the mouthpiece clean, it would be proper and more convenient to have available an inhalator which can be disposed after one single use. Additionally present devices often result in problems of obtaining a proper mixture with the air during the transmission of the medication to the breathing portions of the body thereby affecting efficiency of the medicinal mixture.

But even if it's preferable to use this devices just once, the invention that is object of this patent has been realized in two embodiments: the first one is a disposable inhaler, while the second one can be used more times.

Clinical practice patients, who are often elderly people, find it difficult to understand the multi-disk method of loading and administering the dry powdered medicament. The illustrative page enclosed with the medicine, though clear, is nevertheless only theoretical. In synthesis everything is seen as complex and difficult and is thus refused. There is the actual possibility that the medicine is not administered because the patient has not noticed that the loader is empty after several doses. Then there are hygienic needs to clean the mechanism with the special brush, etc.

All this frequently results in the premature abandoning of the medicinal formulation, possibly to return to the spray. This, despite its apparent simplicity of use and rapidity, is liable to constant errors, both on the part of the patient and on the part of the nurses, so the spray is almost always delivered on the tongue and not in the lower respiratory tract, leading to repeated doses, with possible cumulative side effects.

The serious matter is that the dry powder preparations are almost unknown even in the hospital environment, where the priority in potency and dignity of the "pill" medicine still exists, as it does outside the hospital.

### Disclosure of invention

The elimination of these obstacles which compromise the use of a decisive weapon such as the one object of this invention, is thus desirable. The development of the ***Mono-dose Inhaling Device*** proposed is needful, to properly dispense powered medicines, and for other similarly interesting applications with potentially enormous developments which can be hypothesized for other substances such as:
- Agents useful for calcium regulation (e.g., calcitonin, parathyroid hormone, and the like):
   - Analgesic/antipyretics (e.g., aspirin, acetaminophen, ibuprofen, naproxen sodium, buprenorphine hydrochloride, propoxyphene hydrochloride, propoxyphene napsylate meperidine hydrochloride, hydromorphone hydrochloride, morphine sulfate, oxycodone hydrochloride, codeine phosphate, dihydrocodeine bitartrate, pentazocine hydrochloride, hydrocodone bitartrate, levorphanol tartrate, diflunisal, trolamine salicylate, nalburphine hydrochloride, mefenamic acid, butorphanol tartate, choline salicylate, butalbital, phenyltoloxamine citrate, diphenhydramine citrate, methotrimeprazine, cinnamedrine hydrochloride, meprobamate, and the like);
   - Antianginal agents (e.g., beta-adrenergic blockers, calcium channel blockers (e.g., nifedipine, diltiazem hydrochloride, and the like), nitrate (e.g., nitroglycerin, isosorbide dinitrate, pentaerythritol tetranitrate, erythrityl tetranitrate, and the like);
   - Antidepressant (e.g., doxepin hydrochloride, amoxapine, trazodone hydrochloride, amitriptyline hydrochloride, maprotiline hydrochloride, phenelzine sulfate, desipramine hydrochloride, nortriptyline hydrochloride, tranylcypromine sulfate, fluoxetine hydrochloride, imipramine hydrochloride, imipramine pamoate, nortriptyline, amitriptyline hydrochloride, isocarboxazid, desipramine hydrochloride, trimipramine maleate, protriptyline hydrochloride, and the like);
   - Antianxiety agents (e.g., lorazepam, buspirone hydrochloride, prazepam, chlordiazepoxide hydrochloride, oxazepam, chlorazepate dipotassium, diazepam, hydroxyzine pamoate, hydroxyzine hydrochloride, alprazolam, droperidol, halazepam, chlormezanone, and the like);
   - Antiharrythmics (e.g., bretylium tosylate, esmolol hydrochloride, verapamil hydrochloride, amiodarone, encainide hydrochloride, digoxin, digitoxin, mexiletine hydrochloride, disopyramide phosphate, procainamide hydrochloride, quinidine sulfate, quinidine gluconate, quinidine polygalacturonate, flecainide acetate, tocainide hydrochloride, lidocaine hydrochloride, and the like);
   - Antiarthritic agents (e.g., phenylbutazone, sulindac, penicillamine, salsalate, piroxicam, azathioprine indomethacin, meclofenamate sodium, gold sodium thiomalate, ketoprofen, auranofin, aurothioglucose, tolmetin sodium and the like);
   - Antibacterial agents (e.g., amikacin sulfate, aztreonam, chloroamphenicol, chloramphenicol palmitate, chloamphenicol sodium succinate, cirpofloxacin hydrochloride, clindamycin hydrochloride, clindamycin palmitate, clindamycin phosphate, metronidazole, metronidazole hydrochloride, gentamicin sulfate, licomycin hydrochloride, tobramycin sulfate, vancomycin hydrochloride, polymyxin B sulfate, colistimethate sodium, colistin sulfate and the like);
   - Anticoagulants (e.g., heparin, heparin sodium, warfarin sodium, and the like);
   - Anticonvulsants (e.g., valproic acid, divalproate sodium, phenytoin, phenytoin sodium, clonazepan, primidone, phenobarbitol, phenobarbitol sodium, carbamazepine, amobarbital sodium, methsuximide, metharbital, mephobarbital, mephenytoin, phensuximide, paramethadione, ethotoin, phenacemide, secobarbitol sodium, clorazepate dipotassium, trimethadione, and the like);
   - Antidepressant (e.g. doxepin hydrochloride, amoxapine, trazodone hydrochloride, amitriptyline hydrochloride, maprotiline hydrochloride, phenelzine sulfate, desipramine hydrochloride, nortriptyline hydrochloride, tranylcypromine sulfate, fluxetine hydrochloride, doxepin hydrochloride, imipramine hydrochloride, imipramine pamoate, nortriptyline, amitriptyline hydrochloride, isosarboxazid, desipramine hydrochloride, trimipramine maleate, protriptyline hydrochloride, and the like);
   - Antifibrinolytic agents (e.g., aminocaproic acid);
   - Antifungal agents (e.g., griseofulcin, keloconazole, and the like);
   - Antigout agents (i.e., colchicine, a'iopurinol and the like);
   - Antihypertensive agents (e.g., trimethaphan camsylate, phenoxybenzamine hydrochloride, pargyline hydrochloride, desertpidine, diazoxide, guanethidine monosulfate, minoxidil, rescinnamine, sodium nitroprusside, rauwolfia serpentina, alseroxylon, phentolamine mesylate, reserpine, and the like);
   - Anti-infectives (e.g., GM-CSF);
   - Antimanic agents (i.e., lithium carbonate);
   - Antimicrobials (e..g., cephalosporins (e.g., cefazolin sodium, cephradine, cefaclor, cephapirin sodium, ceftisoxime sodium, cefoperazone sodium, cefotetan disodium, cefutoxime azotil, cefotaxime sodium, cefadroxxil monohydrate, ceftazidime, cephalexin, cephalothin sodium, cephalexin hydrochloride monohydrate, cefamandole nafate, cefoxitin sodium, cefonicid sodium, ceforanide, ceftriaxone sodium, ceftazidime, cefadroxil, cephradine, cefuroxime sodium, ceforanide, ceftraxone sodium, ceftazidime, cefadroxil, cephradine, cefuroxime sodium and the like), penicillins (e.g., ampicillin, amoxicillin, penicillin G benzathine, cyclacillin, ampicillin sodium, penicillin G potassium, penicillin V potassium, piperacillin sodium, oxacillin sodium, bacampicillin hydrochloride, cloxacillin sodium, ticarcillin disodium, azlocillin sodium, carbenicillin indanyl sodium, pinicillin G potassium, penicillin G procaince, methicillin sodium, nafcillin sodium, and the like), erythromycins (e.g. erythromycin ethylsuccinate, erythromycin, erythromicin estolate, erythromycin lactobionate, erythromicin siearate, erythromicin ethylsuccinate, and the like) tetracycline (e.g., tetracycline hydrochloride, doxycycline hyclate, minocycline hydrochloride, and the like), and the like);
   - Antimigraine agents (e.g., erotamine tartrate, propanolol hydrochloride, isometheptene mucate, dichloralphenazone, and the like);
   - Antinauseant/antiemetics (e.g., meclizine hydrochloride, nabilone, prochlorperazine, dimenhydrinate, promethazine hydrochloride, thiethylperazine, scopolamine, and the like);
   - Anti-oxidants (e.g., beta-carotene, butylated hydroxynisole, butylated hydroxytoluene, catalases, coenzyme Q10, glutathione, copper sebacate, folic acid, manganese, retinol, pycnogenol, selenium, superoxide dismutase, lycopene, lipoic acid, acetyl-1-carnitine, N-acetyl cysteine, linoleic acid, vitamins A, B2, B6, B12, C and E, taurine, zinc, adenosine, allicin, aloe, alpha lipoic acid, BHA, BHT, bilirubin, capsaicin, catechin, cysteine, coumarin, curcumin, dimethylglycine, glycine, ferrous fumarate, genistein, ginger, ginkgo biloba, gallates, gluconate, green tea, isoascorbic acid, L-glutamine, L-methyl methionine, L-seleno cysteine, L-seleno methionine, lutein, melatonin, methionine reductase w(Cu-Zn or Mn), N-acyl 1-cysteine esters, N-acyl 1-methionine esters, poplar bud, procyanidin, pycnogenol, resveratrol, rosmary, rutin, rutinose, selenium-yeast, seleno cysteine, seleno methionine, silybum marianum, sodium bisulfite, sodium metasulfite, sodium sulfite, sodium thiosulfite, spirulina, sulfuraphane, superoxide dismutase (SOD), taurine, thioglycerol, thiol, thiosorbitol, thiourea, wheat grass, zinc gluconate and the like);
   - Antiparkinson agents (e.g., ethosuximide, and the like);
   - Antiplatelet agents (e.g., aspirin, empirin, ascriptin, and the like);
   - Antistamine/antipruritics (e.g., hydroxyzine hydrochloride, diphenhydramine hydrochloride, chlorpheniramine maleate, brompheniramine maleate, cyproheptadine hydrochloride, terfenadine, clemastine fumarate, triprolidine hydrochloride, carbinoxamine maleate, diphenylpyraline hydrochloride, phenindamine tartrate, azatadine maleate, tripelennamine hydrochloride, dexchlorpheniramine maleate, methdilazine hydrochloride, trimprazine tartrate, and the like);
   - Antipsychotic agents (e.g., haloperidol, loxapine succinate, loxapine hydroxhloride, thioridazine, thioridazine hydrochloride, thiothixene, fluphenazine hydrochloride, fluphenazine decanoate, fluphenazine enanthate, trifluoperazine hydrochloride, chlorpromazine hydrochloride, perphenazine, lithium citrate, prochlorperazine, and the like);
   - Antiulcer/antireflux agents (e.g., famotidine, cimetidine, ratitidine hydrochloride, and the like);
   - Antiviral agents (e.g., interferon gamma, zidovudine, amantadine hydrochloride, ribavirin, acyclovir, and the like);
   - Bronchiodialators (e.g., sympathomimetics (e.g., epinephrine hydrochloride, metaproterenol sulfate, terbutaline sulfate, isoetharine, isoetharine mesylate, isoetharine hydrochloride, albuterol sulfate, albuterol, bitolterol, mesylate isoproterenol hydrochloride, terbutaline sulfate, epinephrine bitartrate, metaproterenol sulfate, epinephrine, epinephrine bitartrate), anticholinergic agents (e.g., ipratropium bromide), xanthines (e.g. aminophylline, dyphylline, metaproterenol sulfate, aminophylline), mast cell stabilizers (e.g., cromolyn sodium), inhalant cortisteroids (e.g., flurisolidebeclomethasone dipropionate, beclomethasone dipropionate monohydrate), salbutamol, beclomethasone dipropionate monohydrate), salbutamol, beclomethasone dipropionate (BDP), ipratropium bromide, budesonide, ketotifen, salmeterol, xinafoate, terbutaline sulfate, triamcinolone, theophylline, nedocromil sodium, metaproterenol sulfate, albuterol, flunisolide, and the like);
   - Hemorheologic agents (e.g., pentoxifylline);
   - Hypoglycemic agents (e.g., human insulin, purified beef insulin, purified pork insulin, glyburide, chlorpropamide, glipizide, totalbutamide, tolazamide, and the like);
   - Hypolipidemic agents (e.g. clofibrate, dextrothyroxine sodium, probucol, lovastatin, niacin, and the like);
   - Hormones (e.g., androgens (e.g., danazol, testosterone cypionate, fluoxymesterone, ethyltostosterone, testosterone enanihate, methyltesterone, fluoxymesterone, testosterone cypionate), estrogens (e.g., estradiol, estropipate, conjugated estrogens), progestins (e.g.methoxyprogesterone acetate, norethindrone acetate), cortisteroids (e.g. triamcinolone, betamethasone, betamethasone sodium phosphate, dexamethasone, dexamethasone sodium phosphate, dexamethasone acetate, prednisone, methylprednosolone acetate suspension, triamcinolone acetonide, methylprednisolone, prednisolone sodium phosphate methylprednisolone sodium succinate, hydrocortisone sodium succinate, methylprednisolone sodium succinate, triamcinolone hexacatonide, hydrocortisone, hydrocortisone cypionate, prednisolone, fluorocortisone acetate, paramethasone acetate, prednisolone tebulate, prednisolone acetate, prednisolone sodium phosphate, hydrocortisone sodium succinate, and the like), thyroid hormones (e.g., levothyroxine sodium) and the like;
   - Nucleic acids (e.g., sense or anti-nucleic acids encoding any protein suitable for delivery by inhalation, including the proteins described herein, and the like);
   - Proteins (e.g., DNase, alginase, superoxide dismutase, lipase, and the like);
   - Sedatives/hypnotic (e.g., ergotamine tartrate, propanol hydrochloride, isometheptene mucate, dichloralphenazone, and the like);
   - Thrombolytic agents (e.g., urokinase, streptokinase, altoplase, and the like);
Additional agents contemplated for delivery employing the invention inhalation device and methods described herein include agents useful for the treatment of diabetes (e.g., activin, glucamon, insulin, somatostatin, proinsulin, amylin, and the like), carcinomas (e.g., taxol, interleukin-1, interleukin-2, (especially useful for treatment of renal carcinoma), and the like, as well as leuprolide acetate, LHRH analogs (such as nafarelin acetate), and the like, which are especially useful for the treatment of prostatic carcinoma), endometriosis (e.g., LHRH analogs), uterine contraction (e.g., oxytocin), diuresis (e.g., vasopressin), cystic fibrosis (e.g., Dnase (i.e., deoxyribonuclease), SLPI, and the like), neutropenia (e.g., GCSF), lung cancer (e.g., beta 1-interferon), respiratory disorders (e.g. superoxide dismutase), RDS (e.g., surfactants, optionally including apoproteins), and the like.

Presently preferred indications which can be treated employing the invention inhalation device and methods described herein include diabetes, carcinomas (e.g., prostatic carcinomas), bone disease (via calcium regulation), cystic fibrosis and breathing disorders (employing bronchodilators), and the like.

Accordingly, there exists a definite need for a tiny disposable dry powder pharmaceutical delivery device that can use many different powdered pharmaceuticals, which can safely stored to maintain stability until dispensing, without any loss of uptake efficiency due to its advanced design, or to excessive absorption within the mouth or throat. There is a further need for such a device to be conveniently disposable without being overly expensive. The present invention satisfies these needs and provides further related advantages.

A further advantage of the device of the present invention may be represented by an additional protective factor constituted by reduction of oral mycosis, notoriously correlated with the fact that the spray is nearly always deposited in the area of the tongue and oropharynx, also as a result of the backwards direction of the spray itself. This is eliminated with the optimal micronization obtained with the disposable method.

The clinical, pharmacological, economic and finally the social advantages can be anticipated together with the co-operation of the patient (it is sufficient to consider the reduction of anxiety due to the easiness to use the device, the fact that the patient no longer needs the assistance of members of the family, with the reduction of injections, and with lower hospitalization required, etc.).

From studies made by the inventors and from specialist practitioners, as well as from the exchanging of experiences with general practitioners who are called to put into practice the prescriptions of the pneumologist, the therapy with such deficient multi-dose inhalators tends to limit itself, whereas it would be desirable to consider it as a separate and autonomous entity as compared to the aerosol which is often confused with the ready-to-use type. In fact from this point of view it is overtaken by an old systemic medicines (theophylline) which thanks to their necessary regular use space from the B2-stimulants, because they manage to impose a constant and observed rule which is easy to understand.

Therefore the desire of the specialist, but also and especially of the general practitioners to have these potent and precise arms available springs from the above, but with less complexity of use, in order to widen the base of subjects who are the potential users and beneficiaries.

Theoretically possibilities spring from analytical reflection and although they come from someone who does not by profession program company strategies and methods, they offer nevertheless serious and concerned criticism.

In my opinion these solutions are represented by the total upending of the concept of administration, where first of all the interest compliance and serenity of the patient is favored (many tell of anxiety in complex manoeuvres) where he is called to execute the minimum number of actions possible and with a strong temporal and material connotation, i.e. with a precise identification of the act performed in the same way as the taking of any pill, with the result of the exact perception of the completed event (let us not forget that the patient often doubts that he has correctly administered the medicine via aerosol). Consequently there is a strong concentration of faith in the act itself, which as well as scientific becomes "magical", therefore saving and above all worthy of faithfulness.

The regular imposition of the right doses is not the least of the advantages of the methodologies in question. The possibility of lower production costs and the better perception of results by the prescribing doctor, and hence the increase of confidence in the medicinal formulation, is another advantage of the methodology which cannot be ignored.

The use of the breath-activated inhaler of the present invention is extremely simple, fool-proof, and the user will easily achieve maximum control of his health, instead the more complicated models available on the market, anyone using them will be given extensive training for its use. Nevertheless it is generally estimated that with the standard inhaler only about ten per cent (10%) of the inhaler drug actually reaches the lungs, the remaining ninety per cent (90%) is deposited on the lining of the mouth and throat, and when relief is not forthcoming, the inhaler use tends to quick press for another dose of the drug. Using such inhalers to often is likely, under certain circumstances to have serious unpleasant side effects, such as mild irritation in the throat, dysphonia, nausea, jitteriness, indigestion, gastric reflux, insomnia, thrush, hoarseness, coughing, oropharyngeal candidasis, etc.

The use of the *Mono-dose Inhaler* of the present invention through the respiratory tract may provide an efficient means of administering dry powder medicaments such as vasoconstrictors, antihistaminics, antispamodics, antipeptics, antibronchiolitis, B2-stimulants-corticosteroids, antivirals, antifungals, antioxidants, antileukotrienes, antiallergens, antibiotics, human proteins, peptides, etc., which will generally requires a smaller dosage then would be necessary if the drug were given systematically, infection is often accompanied by one or more of the five other conditions cited above.

The principal purpose of the present invention is to provide a ***Mono-dose Inhaling Device*** for dispensing only one dose of powdered medicaments into the *Respiratory Tract* and is intended to be easy to use, inexpensive and then be thrown away when it has served its purpose, even if, in a second embodiment of this invention, it can be used more times.

The present invention relates to a *Mono-dose Device* suitable for delivery, via the respiratory tract, therapeutic medication and includes among its objects and advantages increased convenience in medication with dry medicaments in powder form, especially with respect to accuracy of dosage and accurate placement of the drug.

The inhaler object of this invention is useful also with antibiotics, steroids and other difficulty soluble compounds. Problems associated with the formulation of this drug include aggregation, caking, particle-size growth and often clogging, which using the present *Inhaler* is completely avoided.

It has been discovered that the use of the Inhaler of the present invention for dispensing powdered medicaments available in the form of fine powders, can be practiced to cause effective therapeutic effect. It is also possible to combine the general treatment thus resulting with high local concentration of the same medicament in the respiratory tract.

Solid inhalation therapy is of value in connection with many therapeutic agents, including antiseptic bronchiolytics, and vaso-constrictors, and is indicated for at least some of the known anti-histamine drugs.

Many therapeutic agents which are water soluble, will be found effective by solid inhalation, by both the effect and the degree of effectiveness need to be established by specific test in connection with each substance.

Many ingredients can be added as Membrane Permeation Inhaler to the Drug formulations to increase their barrier permeability, but the preferred one and most effective is *lactose*.

Accordingly, the present invention will succeed in obtaining such constancy by providing a device which is to be utilized by the patient by breathing in, in a simple, substantially normal way. Utilized the energy of the air flowing through the device to deliver into the lining a single dose of powdered medicaments, which charge is delivered quickly, shortly after inhalation begins, and finds its way to its resting place. Thus the device itself and the body passages first receiving the stream of air are thoroughly swept and scavenged with pure air during a major portion of the breathing-in process. I have found that administration in this way not only contributes to deep penetration of the medicament, but that it becomes unnecessary to pulverize or micronize the medicament into a true smoke. The use of larger particle tends to increase the reliability with which a uniform fraction of the material will pass on through the body passages first receiving the stream without getting caught on the moist walls.

With the *Mono-dose Inhaler* of the present invention the absorption is virtually as rapid as the drug can be delivered into the alveoli of the lungs, since the alveolar and vascular epithelial membranes are quite permeable, blood flow is abundant, and there is a very large surface for absorption.

It is an object of this invention to provide an extremely simple device which is sturdy, dependable, reliable, fool-proof and which will maintain its characteristics for a long period so that if a considerable time about elapse between the original manufacture of the item and its utilization, the therapeutic efficacy of the drug will remain comparatively undiminished.

The invention makes possible to dispense any type of powdered drugs which are at all times completely under the control of the user. It will thus be seen that the invention provides a simple and convenient means of dispensing powdered drugs, and it is capable of extremely wide applications.

The *Mono-dose Inhaling Device* of this invention can be manufactured quite easily using means of injection moldings well known in the art, thereby affecting substantial cost reduction in manufacturing the device without adversely affecting the medicament administration inhalation.

With this and other objects in view the invention consists in the details of an inexpensive but efficient inhalator, convenient and easy to use and of low cost to the user so that the device may be discarded following the single administration of the contained medicament.

As can you see from the drawings included, the concept in question is carried out through the use of disposable administration devices which can be adapted in their dimensions, external aspect and internal and external conformation: however, all the devices follow the logic of a single and exact dose of medicine in a container made of plastic, or other appropriate material which can also be biodegradable, and suitable for inhalation, thanks to its conformation.

A Mono-dose inhaling device for dispersing powdered medicaments through the respiratory tract, according to the invention, comprises:
- means to hold a tablet and release the powder it contained, with a body (I) consisting of a horizontal chamber (15), equipped with an opening (8) at one end, and a vertical chamber (9), at the opposite end, which is connected and perpendicular to the horizontal chamber (15), said body (1) being closed at the bottom by a base (14) blocking it;
- means to hold the tablet inside said body (1), with an open housing portion (10), where the tablet (2) is placed, resting on the circular groove (11) of said housing portion (10);
- means to hold the pieces of the case of the tablet (2), after it has been split, and to prevent the powder from aggregating, with a reservoir (5) having a series of slots on its walls that hold the bigger pieces inside, while letting the powder pass through;
- means to close the body (1) comprising the tablet, with a cover member (4), which enters the vertical chamber (9) and has two outer protrusions (16, 16A) on its walls that stop under the inner protrusions (17, 17A) of the chamber (9), preventing the cover from accidentally escaping from its place;
- means to allow the cover (4) to go downwards and upwards till the stop of the protrusions, with a spring (3) inside the chamber (9) that touches the outer edge of the housing portion (10) on its lower side and the inner surface of the cover on its upper side;
- means to split the tablet (2), with said cover (4) having inside a pointed element (12) that passes through the spring (3); when the cover (4) is pressed, it perforates the tablet (2) placed on the housing portion (10), dispersing its powder into the chamber (15) and holding the pieces of the case in the reservoir (5);
- means to deliver the powder of the tablet released into the body (1), with an opening (8) from which the user can inhale the powder.

Conveniently, the body of the inhaler is formed by two parts connected each other: the body (1) and the base (14), besides a cover member (4) that enters the chamber (9). Such a conformation facilitates the manufacturing and assembling of this device, considerably reducing the costs of production.

Conveniently, in order to prevent the tablet from escaping from its place and to ensure a greater stability of the spring, an element (3A) is placed over the tablet, consisting of a circular bend resting on the outer edge of the housing portion (10) and a central hole allowing the pointed end (12) to pass through.

Conveniently, under the housing portion (10) of the tablet, a reservoir (5) is provided, with a series of slots on its walls and a grid shape, which allows the pieces of the case of the tablet to remain inside the reservoir. Said reservoir has the important function to prevent the powder from aggregating in a humid atmosphere, obstructing in this way its dispersion into the chamber. The powder, which is filtered by the reservoir (5), passes into the chamber (15), while the conformation of the reservoir facilitates the creation of a vortex of air inside the device and the consequent easy inhalation of the powder itself.

Conveniently, the entrance nozzle of the air is supplied by a special valve which ensures the one-way flow of the air and eliminates the possibility of mistaken actions before or during the inhalation of the powdered medicament.

Conveniently, in order to filter possible pieces of the case inside the horizontal chamber (15), a vertical grid (7) can be additionally provided.

Conveniently the cover member (4) and the vertical chamber (9) where it enters, have a square section and a parallelepiped shape.

Conveniently, the cover member (4) has some grooves, vertical or others, on its side surfaces, so as to ensure a better air-flow and thus facilitate the inhalation of the powder.

Conveniently, said cover member (4), on the base of at least two opposite side surfaces, has one or more protrusions (16, 16A) that stop under the upper inner protrusions of the chamber (9).

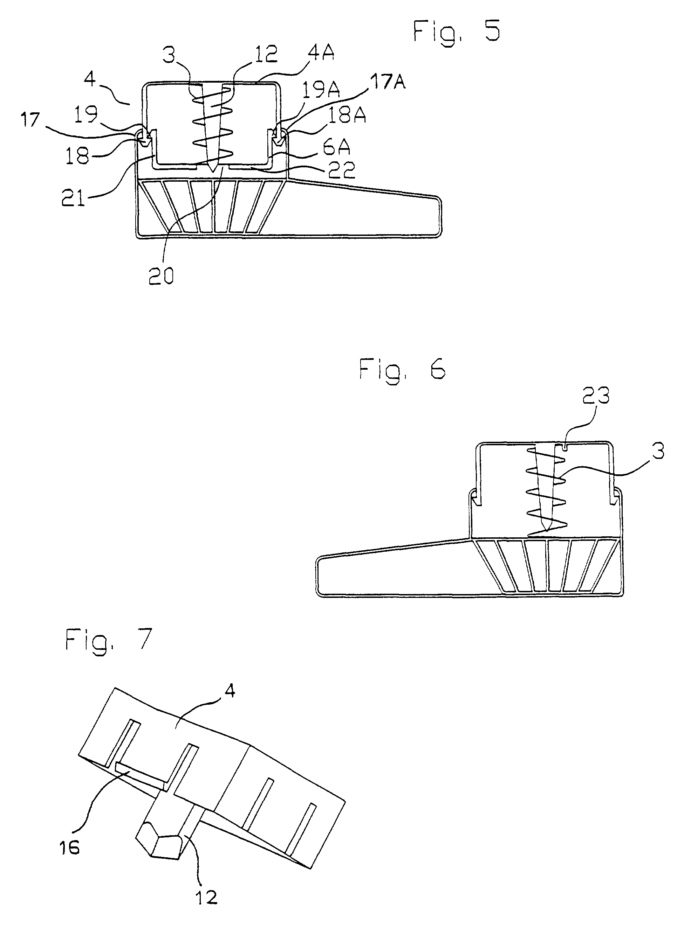

Conveniently, the foil (12) inside the cover has a blunt point (Fig. 7), so as to increase the impact surface of the foil against the tablet, ensuring its perfect split.

Preferably, with reference to Figs, 1, 2, 3 and 4, the inhaler is mono-dose, even if it can be used more times also in this manufacturing embodiment.

Conveniently, with reference to Fig. 5, the inhaler has a cover member (4), comprising a pointed end (12) and a spring (3), which touches the inner surface of the upper part (4A) on the top, and the inner surface (22) of the lower part (21) that runs inside the part (4A).

Conveniently, the spring (3) is made of metal, plastic or other suitable material.

Conveniently, with reference to Fig. 5, the lower part (21), on its base (22), has a central hole that lets the foil (12) to pass through.

Conveniently, with reference to Fig. 5, by pressing the cover (4), its upper part (4A) runs downwards pressing the spring (3) and making the foil (12) perforate the tablet (2); by releasing the cover (4), it stops upwards where its external upper protrusions (18, 18A) meet the internal protrusions (17, 17A) of the chamber (9).

Conveniently, with reference to Fig. 5, the cover (4) can be totally pulled out from the chamber (9) keeping the spring (3) inside and preventing the other elements from dissipating, so permitting to substitute the old tablet with a new one.

Conveniently, with reference to Fig. 6, the spring (3) is connected inside the cover (4) by a slightly protruding hinge (23) placed on the top. This solution allows the cover (4) to be pulled out from the chamber (9) keeping the spring (3) inside, so permitting to substitute the old tablet with a new one.

Conveniently, the powder released in the body (1) of the device is inhaled by the user by placing the outer end of the chamber (15), opposite to the cover member (4), into his/her mouth and breathing in, so that the powder coming out from the opening (8) enters the mouth and reaches the lungs.

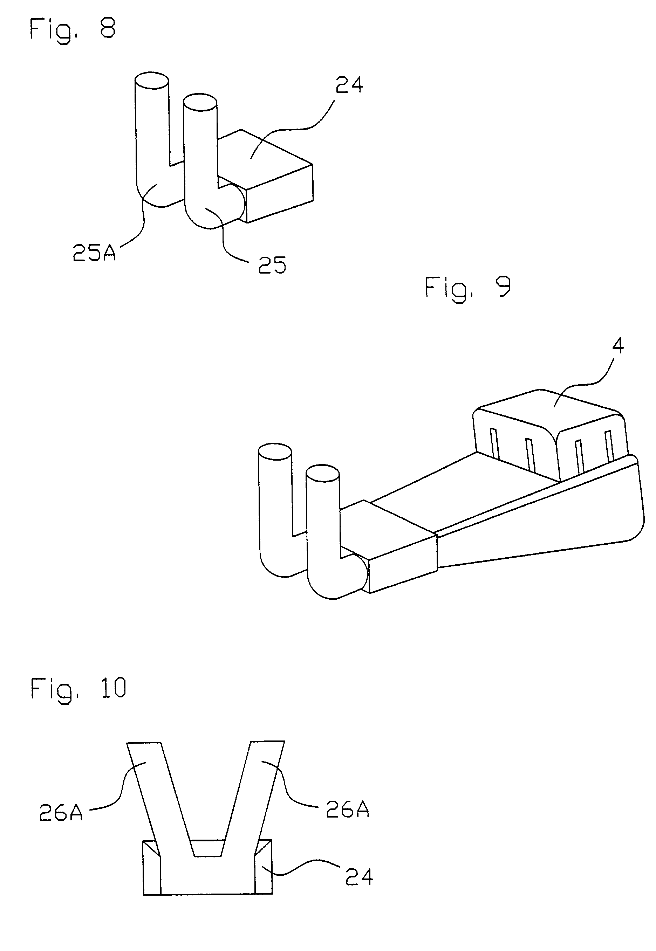

Conveniently, with reference to Figs. 8, 9 and 10, the inhaler may be equipped with accessories allowing it to be used even by inhaling with one's nose. In particular, an accessory is provided, with a connecting element (24) to be inserted into the opening (8), equipped with two vertical (25, 25A) or oblique (26, 26A) ducts that the user put into his/her nostrils, inhaling the substance by the nose.

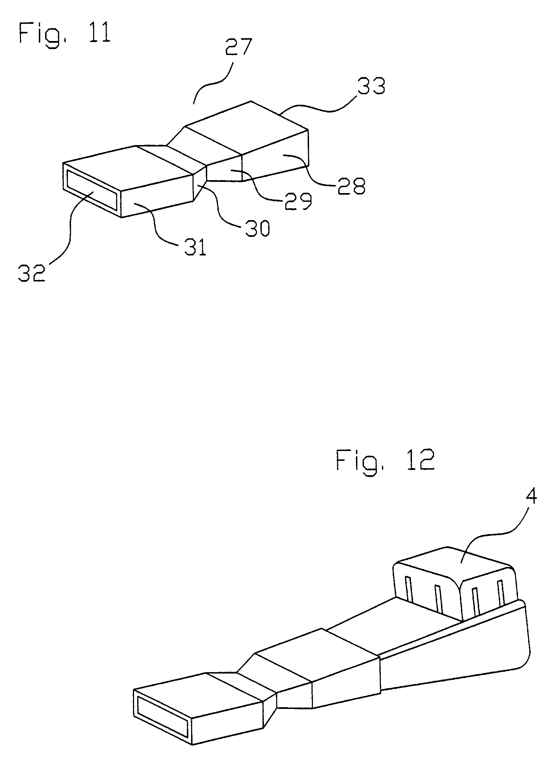

Conveniently, with reference to Figs. 11 and 12, the device may be equipped with an accessory permitting to deeply inhaling the powdered substances contained into the tablet. Particularly, this accessory consists of two chambers: one (28) has an opening that enters the opening (8) of the device, while the other (32) is used to inhale the substance.

Conveniently, with reference to Figs. 11 and 12, this accessory has a central tightening (29, 30) between the two chambers, so that a vortex of air rises inside, in order to facilitate the inhalation of the powder.

The operation of the invention is thought to be obvious from the above description of the structure of the various forms of the device, and it is considered unnecessary to enumerate the various substances which can be conveniently dispersed in this manner, since any substance which is in powdered form will exhibit the desired results.

Furthermore to obviate the above described disadvantages of the Inhaler of the prior art, the inventor developed the present *"Mono-dose Device to efficiently dispense powdered Medicaments through the Respiratory Tract"*, providing the following advantages:
- Easy to use, relieving the patient of mental tension and anxiety regarding the mode of administration and the immediate check of the completed operation.
- Placebo effect advantage for the Patient deriving from the psychological tranquilization due to the simplicity of the method of administration, this being particularly relevant on old age patients.
- Guarantee of the correct dosage and achievement of the therapeutic result, not only to benefit illiterate and elderly patients, but children too.
- Optimum of the dispensing method due to its simplicity, dependability, reliability, fool-proof, and manufacturing low cost.
- Will be discarded following the administration of the contained drug, avoiding the hygiene problems of the Multi-dose Devices which after the first time of its use can be bacteria contaminated and are easily soiled and subject to blockage.
- Elimination of various Doctors reservations, which currently are not very much in favor to preserve extensively the use of powdered drugs through the use of inhalers.
- Avoiding patient education and extensive training in how to use them, particularly for chronic therapies (many patients have difficulty with inhalers that require more than one step).
- Efficacious and ready to be used with most powdered medicines, just to mention few but not limited to: antibiotics, mucolitics, antioxidants, hormone, vaccines, corticosteroids, etc., which are still not prescribed via inhalations due to lack of efficient devices and cultural instruction.
- Elimination of the confusion between long-acting and ready-to-use medicines.
- Resolution of the problem of patient co-operation.
- Eye appealing and very small.

Moreover, the *Mono-dose Device* in question eliminates manual problems, problems of maintenance, hygiene, comprehension, mistaken use and psychological conditions, and there is no doubt that the *Disposal Device* of the present invention is the most uncomplicated and efficient method available for delivery powdered medicaments via the respiratory tract.

On the basis of what is stated, considering that the *Mono-dose Device* is specifically dedicated to clinical use in the administration of powdered medicines which carry out their action locally on the respiratory tract or through the lung as noninvasive route of administration to the systemic circulation, and it is held that this mechanism is capable of eliminating difficulties and uncertainty due to current methods of administration.

### MONO-DOSE INHALING DEVICE PERFORMANCE DATA TEST

According to the present state of the art for testing the efficiency of inhalers, I carried out representative tests comparing the Device of the present invention with several inhalers of the prior art.

My research for the experimental test has included basic scientific foundation of inhalation and applied aerosol science, comparative respiratory tract anatomy and physiology, inhalation exposure technique, selection of endpoints including applicable regulations and guidelines previously published by scientific literature to find a simple inhalation testing procedure to fulfill the assigned tasks to evaluate the efficiency of the *Inhaling Device* and to provide a foundation of my invention.

The result ascertained by experiments of the rate of powdered drug scattered for the inhalers are shown in Table 1.

**Table 1**

| **COMPARISONS OF SCATTERING PROPERTY BETWEEN DRY POWDER INHALERS** | | | | |
|---|---|---|---|---|
| Dry Powder Inhalers (D.P.I.) | # of Units | Rate of Dry Powder remaining in Inhaler (%) | Rate of Dry Powder remaining in Capsule (%) | Rate of Scattered Dry Powder (%) |
| D.P.I. of this invention | 6 | 5.7±1 | 0.3±1 | 94.0±1 |
| D.P.I. of Prior Art | 6 | 18.8±1 | 3.1±1 | 78.1±1 |

As appears evident from Table 1 each of the *Mono-dose Inhaling Device* of the present invention, each has a very excellent scattering performance with very low rate of powdered drug remaining in the Inhalers, compared with the prior art.

While a particular form of the invention has been illustrated and described, it will be apparent that various modifications can be made without departing from the spirit and scope of the invention. Thus, although the invention has been described in detail with reference to preferred embodiments, those having ordinary skill in the art appreciate that various modification can be made without departing from the invention.

It is specified that the appearance and the basis conformation of the object described is indicative, other advantages of the device will appear to those skilled in the art from the containing of the appended drawings.

It will be readily appreciated that the forms of the invention described above are intended for purposes of illustration only, and numerous changes in the details of construction and materials employed may be made without departing from the spirit of the invention or the scope of the appended claims.

### Brief description of drawings

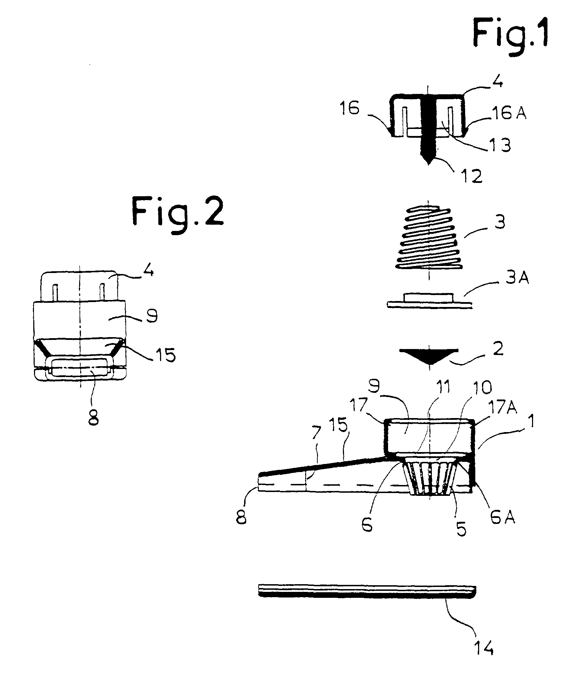

- Fig. 1 is an exploded view of one embodiment of the present inhaler, consisting of a base (14) fixed under the inhaler's body (1), which is equipped with a hollow extended portion (15), with an opening (8) from which the powder can be inhaled, an internal reservoir (5) where the powder falls in, connected to the body by its perimeter (6, 6A), and possibly a small vertical grid (7). Above the reservoir (5), there is an opening (10) and a larger groove (11) where the edge of the tablet (2) rests. This reservoir has a slightly sloping shape and a side surface with a plurality of slots allowing the passage of the powder in a progressive and accurate way during inhalation. Opposite to the opening (8), the body (1) proceeds upwards with a hollow open chamber (9), which has two or more protrusions (17, 17A) on the top, stopping the run upwards of the cover member. Over the tablet (2), which rests on the housing portion (11, 10), there is a support (3A) holding a spring (3). The tablet is slightly pressed by an upper cover member (4), which is hollow inside and has the same shape as the chamber (9) where it's inserted. Said cover member (4) has an internal foil (12) that passes through the spring (3) and perforates the tablet (2), and has two or more protrusions (16, 16A) outside its walls (13), which stop under the protrusions (17, 17A) placed inside the chamber (9). The cover member (4), thanks to its conformation, runs downwards inside the chamber (9), but cannot escape as it is blocked on the top. In this first embodiment, the invention is disposable, therefore, when the cover (4) is pulled out by force, the spring comes out from its portion and it's no more possible to use the inhaler.
- Fig. 2 shows the frontal view of the same object as Fig. 1, with the opening (8) from which the powder can be inhaled, the extended portion (15) of the body (1) and the chamber (9) over which the cover (4) sticks out.

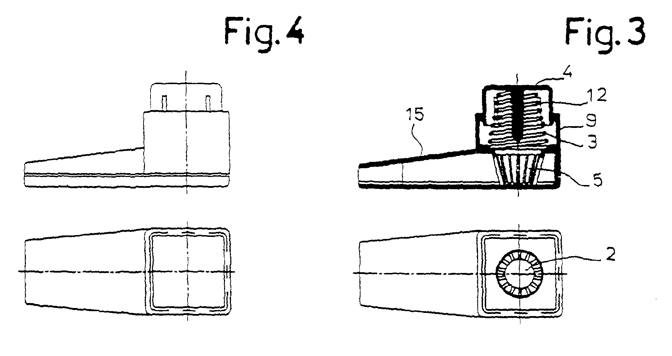

- Fig. 3 shows the side sectional view of the same object as Fig. 1, with the extended portion (15) and the cover (4) inside the chamber (9), comprising the spring (3). This figure shows also the top view of the same object, without the cover (4) and with the tablet over the reservoir (5). It's possible to notice that, by firmly pressing the cover (4) with a finger towards the inside of the chamber (9) to its base, exerting a pressure on the spring, the foil (12) perforates the tablet (2) making the powder come out into the reservoir (5), which holds the pieces of the case and lets the powder disperse in the body of the inhaler, so that the user can inhale it by placing the opening (8) into the mouth and breathing in. As soon as the finger releases the upper body, it comes back in its initial position.
- Fig. 4 shows the same object assembled, both from a side sectional view and a top view.
- Fig. 5 shows a different embodiment of this invention, allowing it to be used more than once, thanks to the cover that can be pulled out from its place for inserting another tablet. Said inhaler has the same characteristics of the previous one, except for the cover (4) that is formed by the same external case (4A) that ends on the base with a double protrusion (18, 18A). The outer part of these protrusions catches the chamber (9), like in the solution above described, while their inner part catches the protrusions (19, 19A) of the upper part of the element (21). Said cover (4) is formed by an upper cover element (4A), from which a foil (12) comes out. Around this foil, there is a spring (3) that rests on the base (21) of the cover. Said base (21) consists of a surface (22) that has a central hole (20) through which the foil (12) passes, and of vertical walls that have two protrusions (19, 19A) on the top, resting on the inner protrusions (18, 18A) of the cover element (4A). In this way, pressing the element (4A) downwards, it runs outside the walls of the base (21) until the foil (12), passing through the hole (20), perforates the tablet. At this moment, releasing the cover element (4A), it runs upwards stopping when its protrusions (18, 18A) touches the hinges (19, 19A) of the element (21).
   In this technical solution, the inhaler can be used more times because it's possible to substitute the old tablet with a new one, by pulling the cover out and keeping the spring inside.
- Fig. 6 shows another embodiment of this invention, which can be used more times, where the spring (3) is stopped on the top by a protrusion (23) placed inside the cover (4). Even in this case, by pulling the cover out after the use, the spring remains inside the cover and a new tablet can be put into the inhaler.
- Fig. 7 is a prospective view of the conformation of the cover member (4) showing the shape of the point of the foil (12).
- Fig. 8 shows an accessory to be applied to the end portion of the inhaler in order to allow the breathing-in of powdered substances by the nose. Said accessory consists of an element (24), preferably made of soft rubber, which is the connecting element with the inhaler; at its opposite end, there are two vertical ducts (25, 25A) that must be inserted into the nostrils.
- Fig. 9 shows the same accessory as Fig. 8 applied to the inhaler in order to breathe in by the nostrils, always pressing the cover (4).
- Fig. 10 shows the same accessory as Fig. 8, with the two ducts (26, 26A) that diverge from the connecting element (24) taking an oblique position.
- Fig. 11 shows a further accessory of the inhaler, suitable for the delivery of powders that must be inhaled deeply. It consists of an element (27) formed by a connecting part (28) with an opening into which the inhaler's opening (8) is inserted. Said part (28) ends with a tightening (29) that proceeds with a reverse further tightening (30) and then an enlarged body (31) with an opening from which the powdered substance can be inhaled. The particular conformation of this accessory and its tightening at the center of the body, permit the creation of a sort of vortex of air that facilitates the inhalation of the powder.
- Fig. 12, finally, shows the same accessory as Fig. I I applied to the inhaler in order to deeply breathe in substances, always pressing the cover (4).

## Claims

1. A mono-dose inhaling device for dispersing powdered medicaments through the respiratory tract, according to the invention, **characterized by** the fact that it comprises:
- means to hold a tablet and release the powder it contained, with a body (I) consisting of a horizontal chamber (15), equipped with an opening (8) at one end, and a vertical chamber (9), at the opposite end, which is connected and perpendicular to the horizontal chamber (15), said body (1) being closed at the bottom by a base (14) blocking it;
- means to hold the tablet inside said body (1), with an open housing portion (10), where the tablet (2) is placed, resting on the circular groove (11) of said housing portion (10);
- means to hold the pieces of the case of the tablet (2), after it has been split, and to prevent the powder from aggregating, with a reservoir (5) having a series of slots on its walls that hold the bigger pieces inside, while letting the powder pass through;
- means to close the body (1) comprising the tablet, with a cover member (4), which enters the vertical chamber (9) and has two outer protrusions (16, 16A) on its walls that stop under the inner protrusions (17, 17A) of the chamber (9), preventing the cover from accidentally escaping from its place;
- means to allow the cover (4) to go downwards and upwards till the stop of the protrusions, with a spring (3) inside the chamber (9) that touches the outer edge of the housing portion (10) on its lower side and the inner surface of the cover on its upper side;
- means to split the tablet (2), with said cover (4) having inside a pointed element (12) that passes through the spring (3); when the cover (4) is pressed, it perforates the tablet (2) placed on the housing portion (10), dispersing its powder into the chamber (15) and holding the pieces of the case in the reservoir (5);
- means to deliver the powder of the tablet released into the body (1), with an opening (8) from which the user can inhale the powder.

2. A mono-dose inhaling device according to claim 1, **characterized by** the fact that its body is formed by two parts connected each other: the body (1) and the base (14), besides a cover member (4) that enters the chamber (9). Such a conformation facilitates the manufacturing and assembling of this device, considerably reducing the costs of production.

3. A mono-dose inhaling device according to claim 1, **characterized by** the fact that, in order to prevent the tablet from escaping from its place and to ensure a greater stability of the spring, an element (3A) is placed over the tablet, consisting of a circular bend resting on the outer edge of the housing portion (10) and a central hole allowing the pointed end (12) to pass through.

4. A mono-dose inhaling device according to claim 1, **characterized by** the fact that, under the housing portion (10) of the tablet, a reservoir (5) is provided, with a series of slots on its walls and a grid shape, which allows the pieces of the case of the tablet to remain inside the reservoir, so preventing the aggregation of the powder and facilitating the creation of a vortex of air inside the device and the consequent easy inhalation of the powder itself.

5. A mono-dose inhaling device according to claim 1, **characterized by** the fact that, in order to filter possible pieces of the case inside the horizontal chamber (15), a vertical grid (7) can be additionally provided.

6. A mono-dose inhaling device according to claim 1, **characterized by** the fact that the cover member (4) and the vertical chamber (9) where it enters, have a square section and a parallelepiped shape.

7. A mono-dose inhaling device according to claim 1, **characterized by** the fact that the cover member (4) has some grooves, vertical or others, on its side surfaces, so as to ensure a better air-flow and thus facilitate the inhalation of the powder.

8. A mono-dose inhaling device according to claim 1, **characterized by** the fact that said cover member (4), on the base of at least two opposite side surfaces, has one or more protrusions (16, 16A) that stop under the upper inner protrusions of the chamber (9).

9. A mono-dose inhaling device according to claim 1, **characterized by** the fact that the foil (12) inside the cover has a blunt point, so as to increase the impact surface of the foil against the tablet, ensuring its perfect split.

10. A mono-dose inhaling device according to claim 1, **characterized by** the fact that it's preferably disposable, nevertheless it can be used more times.

11. A mono-dose inhaling device according to claim 1, **characterized by** an inhaler that has a cover member (4), comprising a pointed end (12) and a spring (3), which touches the inner surface of the upper part (4A) on the top, and the inner surface (22) of the lower part (21) that runs inside the part (4A). The element (21), on at least one of the two side surfaces, has two upper protrusions (19, 19A) that rest on two lower internal protrusions (18, 18A) of the part (4A), stopping the run downwards and preventing the lower part (21) from escaping. Said lower part (21), on its base (22), has a central hole that lets the foil (12) to pass through. By pressing the cover (4), its upper part (4A) runs downwards pressing the spring (3) and making the foil (12) perforate the tablet (2); by releasing the cover (4), it stops upwards where its external upper protrusions (18, 18A) meet the internal protrusions (17, 17A) of the chamber (9).

12. A mono-dose inhaling device according to claim 1, **characterized by** an inhaler that has a cover member (4) comprising a spring (3), which is connected to the cover by a slightly protruding hinge (23).

13. A mono-dose inhaling device according to claim 1, **characterized by** the fact that the powder released in the body (1) of the device is inhaled by the user by placing the outer end of the chamber (15), opposite to the cover member (4), into his/her mouth and breathing in, so that the powder coming out from the opening (8) enters the mouth and reaches the lungs.

14. A mono-dose inhaling device according to claim 1, **characterized by** the fact that the entrance nozzle of the air is supplied by a special valve which ensures the one-way flow of the air and eliminates the possibility of mistaken actions before or during the inhalation of the powdered medicament.

15. A mono-dose inhaling device according to claim 1, **characterized by** the fact that it can be manufactured quite easily using means of injection moldings well known in the art, thereby affecting substantial cost reduction in manufacturing the device without adversely affecting the medicament administration inhalation.

16. A mono-dose inhaling device according to claim 1, **characterized by** the fact that the spring (3) is made of steel, plastic or other suitable material.

17. A mono-dose inhaling device according to claim 1, **characterized by** the fact that it may be equipped with or more accessories allowing it to be used even by inhaling with one's nose. In particular, an accessory is provided, with a connecting element (24) to be inserted into the opening (8), equipped with two vertical (25, 25A) or oblique (26, 26A) ducts that the user put into his/her nostrils, inhaling the substance dispersed into the chamber (15) by the nose.

18. A mono-dose inhaling device according to claim 1, **characterized by** the fact that it may be equipped with an accessory permitting to deeply inhaling the powdered substances contained into the tablet. Particularly, this accessory consists of two chambers: one (28) has an opening that enters the opening (8) of the device, while the other (32) is used to inhale the substance. This accessory has a central tightening (29, 30) between the two chambers, so that a vortex of air rises inside, in order to facilitate the inhalation of the powder.
